# EUROPEAN PATENT APPLICATION

(11) **EP 4 016 448 A1**
(43) Date of publication of application: **22.06.2022**
(21) Application number: 20215700.4
(22) Date of filing: 18.12.2020
(51) Int. Cl.: G06T 7/00

(54) **IMAGE ANALYSIS METHOD FOR IMPROVED CLINICAL DECISION MAKING**

(71) Applicant: Oncoradiomics SA, 4000 Liège (BE)
(72) Inventor: VOS, Wim, 9140 Temse (BE); WALSH, Sean, 6255 NN Noorbeek (NL); LEIJENAAR, Ralph T.H., 6436 EA Amstenrade (NL)
(74) Representative: Willnegger, Eva

(57) **Abstract**

The present invention relates to an image analysis method for providing information for supporting diagnostic, therapeutic, prognostic or theragnostic purposes of human and animal bodies. The present invention also relates to a system for performing image-based analysis tasks for providing information for supporting diagnostic, therapeutic, prognostic or theragnostic purposes of human and animal bodies.

## Description

### FIELD OF INVENTION

The present invention relates to the field of image analysis. In particular, the present invention relates to the field of image analysis for predictive medicine and clinical-decision support.

### BACKGROUND OF INVENTION

Medical image analysis allows to extract, from a medical image such as a positron emission tomography (PET) image, a computed tomography (CT) image or a magnetic resonance (MR) image, meaningful image features. Image features are quantitative image metrics that correlate with clinically relevant information, such as a disease stage and histological parameters. Image analysis techniques can therefore be used to obtain predictive, diagnostic, therapeutic or prognostic markers in a safe, non-invasive and longitudinal manner.

Moreover, contrarily to invasive techniques such as biopsies, image analysis allows to assess a patient's lesion as a whole. This is all the more important in oncology, since it is known that intra- and inter- tumor heterogeneity are associated with treatment resistance, treatment failure and poor prognosis. Validated imaging markers are therefore needed to properly assess tumor heterogeneity, to better stratify patients and hence to support clinical decision-making in the era of precision medicine.

Segmentation of the region of interest, such as a neoplasm, is a preliminary step in the process of image features extraction. Manual segmentation is time consuming and user-dependent hence, several automatic and semi-automatic segmentation methods exist to overcome these problems.

Examples of segmentation methods relying on morphological operations and be found in the works of S. Echegaray et al.: "Core samples for radiomics features that are insensitive to tumor segmentation: method and pilot study using CT images of hepatocellular carcinoma" (Journal of Medical Imaging (Bellingham), 2015) and "A Rapid Segmentation-Insensitive "Digital Biopsy" Method for Radiomic Feature Extraction: Method and Pilot Study Using CT Images of Non-Small Cell Lung Cancer" (Tomography, 2016). The former uses a "core sample" segmentation method based on computing the maximal circle inscribed in a manually outlined region and obtaining features from such "core sample", the latter applies a segmentation method based on erosion and dilation.

Examples of semi-automatic segmentation methods that can be applied to medical images are GrowCut, Gaussian mixture models, fuzzy c-means clustering method. These three methods have been compared by H. Veeraraghavan et al. in "Appearance Constrained Semi-Automatic Segmentation from DCE-MRI is Reproducible and Feasible for Breast Cancer Radiomics: A Feasibility Study" (Scientific Reports, 8, 4838, 2018).

Although many segmentation approaches have been developed for extracting quantitative image features from a gross tumour volume (GTV), the predictive models based on the segmentation methods of the prior art are characterized by poor predictive performances, such as for instance poor accuracy, precision, recall, F-score, or AUC values.

The present invention relates to an image analysis method that solves the problems of the prior art. In particular, the present invention provides an image analysis method and a high-performance predictive model for supporting the clinical decision-making process.

### SUMMARY

The present invention relates to an image analysis method for providing information for supporting diagnostic, therapeutic, prognostic or theragnostic purposes of human and animal bodies, comprising the steps of:
- providing, by a processing unit, an image I;
- defining, by said processing unit, a region of interest to obtain a first image shape A and optionally defining, by said processing unit, a second region of interest to obtain a second image shape C;
- modifying, by said processing unit, the first image shape A and optionally modifying the second image shape C so as to obtain a plurality of modified image shapes B;
- deriving, by said processing unit, for each of the image shapes defined in the defining step and obtained in the modifying step, a plurality of image feature parameters; and calculating at least one image feature parameter value from each plurality of image feature parameters;
- optionally selecting, by said processing unit, a subset of image feature parameters from the pluralities of image feature parameters obtained in the derivation step, the subset of image feature parameters being selected based on the at least one image feature parameter value;
- deriving, by said processing unit, using reference data a predictive, diagnostic, therapeutic, prognostic or theragnostic value for supporting medical decision making,
wherein the predictive value is derived based on at least one image feature parameter that is derived from the modified image shape B.

In one embodiment, the modification step comprises shifting of the center of the first imaging shape A and optionally shifting the center of the second image shape C.

In one embodiment, the modified image shape B is obtained by shifting the center of the first image shape A by a shifting distance *a* in any direction a whilst preserving the size and the shape of the image shape A, so as to obtain a modified image shape Ba.

In one embodiment, the shifting distance *a* is comprised between 0 and *a* max, wherein *a* max is the largest shift in a specified direction without any part of the modified image shape Ba extending outside the image I region.

In one embodiment, the modification step comprises resizing the first image shape A and optionally resizing the second image shape C.

Said resizing may be performed by applying a size multiplier b whilst preserving the center position, so as to obtain a modified image shape Bb.

In one embodiment, the resizing multiplier *b* is > 0 and less than *b* max, wherein *b* max is the largest multiplier for which none of the modified image shape Bb extends outside the image region.

In one embodiment, the modification step comprises deforming the first image shape A and optionally deforming the second image shape C.

The deforming may be performed by applying a geometrical deformation multiplier c to obtain a modified image shape Bc.

In one embodiment, the geometrical deformation multiplier c is selected based on the two-dimensional or three-dimensional geometrical shape most closely fitting to the image shape A.

In one embodiment, the modification step comprises: deforming the first imaging shape A and, optionally, deforming the second image shape C by a physiological deformation multiplier *d* to obtain a modified image shape Bd.

The physiological deformation multiplier d may be selected based on the shape and size of an organ or parts thereof.

In one embodiment, the modification step comprises two or more of the shifting, resizing or deformation steps as described here above.

In one embodiment, the modification step comprises at least one morphological operation between two or more of any of the available shape A, shape C and shapes B.

In one embodiment, the present method comprises a derivation step. Said derivation step may comprise: combining a plurality of image feature parameter values derived from the first image A and from the modified image B.

The present invention also relates to a computer program product for providing information supporting diagnostic, therapeutic, prognostic or theragnostic purposes of human and/or animal bodies, the computer program product comprising instructions which, when the program is executed by a computer, cause the computer to carry out the steps of the method described here above.

The present invention also relates a computer-readable storage medium comprising instructions which, when the program is executed by a computer, cause the computer to carry out the steps of the method as described here above.

The present invention also relates to a system for performing image-based analysis tasks for providing information for supporting diagnostic, therapeutic, prognostic or theragnostic purposes of human and animal bodies, the system comprising a processing unit configured to:
- provide an image I;
- define a region of interest in the image I so as to obtain a first image shape A and optionally define a second region of interest so to obtain a second image shape C;
- modify the first image shape A and optionally modify the second image shape C to obtain a plurality of modified image shapes B;
- derive from each of the image shape A, the modified image shape B and optionally second image shape C, a plurality of image feature parameters; and calculate at least one image feature parameter value from each plurality of image feature parameters;
- based on the at least one image feature parameter value, select a subset of image feature parameters from the pluralities of image feature parameters, the subset of image feature parameters comprising at least one image features parameter derived from the modified image shape B;
- using reference data, derive a predictive, diagnostic, therapeutic, prognostic or theragnostic value for supporting medical decision making.

### DETAILED DESCRIPTION

The following detailed description will be better understood when read in conjunction with the drawings. For the purpose of illustrating, the image shapes are therein shown in the preferred embodiments. It should be understood, however that the application is not limited to the precise embodiments shown. The drawings are not drawn to scale and are not intended to limit the scope of the claims to the embodiments depicted. Accordingly, it should be understood that where features mentioned in the appended claims are followed by reference signs, such signs are included solely for the purpose of enhancing the intelligibility of the claims and are in no way limiting on the scope of the claims.

The present invention relates to an image analysis method for providing information for supporting diagnostic, therapeutic, prognostic or theragnostic purposes of human and animal bodies.

The method according to the present intention comprises a step of providing 100, by a processing unit, an image I.

In one embodiment, the image I is a positron emission tomography (PET) image, such as an FDG-PET or an FLT-PET image.

In one embodiment, the image I is a magnetic resonance (MR) image, such as a T1-weighted image, a T2-weighted image, a diffusion-weighted MR image, a dynamic contrast-enhanced (DCE) MR image, a MR spectroscopy image, a dynamic susceptibility contrast (DSC) image, an apparent diffusion coefficient (ADC) map, a functional MR image (fMRI).

In one embodiment, the image I is a computed tomography (CT) image.

In one embodiment, the image I is an X-ray image.

In one embodiment, the image I is an ultrasound image.

The image I could be in any digital format.

In one embodiment, the image I is filtered.

The method according to the present invention comprises a step of defining 200 a region of interest in the image I, so to obtain a first image shape A.

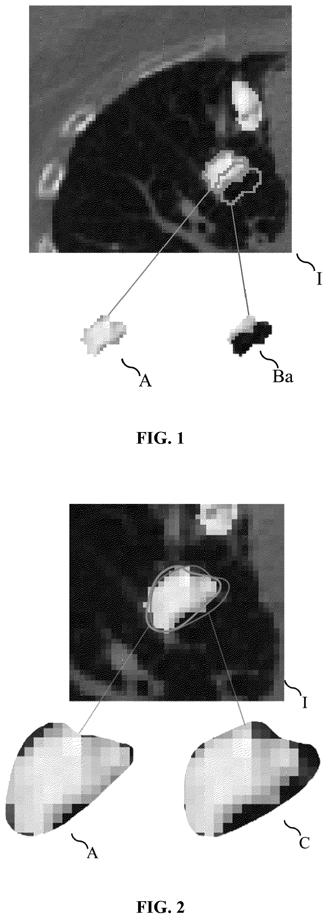

In one embodiment, the step 200 optionally further comprises a step of defining 201 a second region of interest in the image I, so as to obtain a second image shape C. One non-limiting example of this embodiment is illustrated in **figure 1****.** For intelligibility purpose, the images shapes A and C are represented within the image I as well as magnified and isolated from the parent image I.

In one embodiment, the region of interest is defined by a processing unit.

In one embodiment, the processing unit is configured to execute a region growing algorithm in order to define the region of interest.

In one embodiment, the initial seed points of the region growing algorithm are selected by a user.

In one embodiment, the region of interest is hand-drawn.

In one embodiment, the region of interest is any predefined shape such as a circle, an ellipse, a polygon, a rectangle.

In one embodiment, the region of interest is a neoplasm.

In one embodiment, the region of interest is loaded from a memory.

In one embodiment, the region of interest is stored in a memory.

The method according to the present invention comprises a step of modifying 300 the first image shape A, which optionally further comprises a step of modifying 301 the second image shape C, so as to obtain a plurality of modified image shapes B.

In the modification step 300, the image shapes previously obtained are modified by a processing unit.

In one embodiment, the first image shape A is modified so as to obtained one modified image shape B.

In one embodiment, a predetermined number *N* of modifications is applied to the image shape A, so as to obtain N modified image shapes B.

In one embodiment, the modified image shapes B comprise at least one pixel that is overlapped with a pixel of the image shape A.

By overlapped pixels, it is meant pixels that have the same position in the image I.

If a second image shape C is defined, at least one modified image shape B is obtained from the second image shape C. In one embodiment, a predetermined number M of modifications is applied to the image shape C, so as to obtain M modified image shapes B.

The preferred modifications and the corresponding modified image shapes B thereby obtained will be explained hereunder. In one embodiment, the modification step 300 comprises a combination of such modifications.

By convention, each of the modified image shapes B has a reference formed by the letter "B" followed by a character "x". The character "x" indicates the type of modification applied to the image shape A, or optionally to the second image shape C, in order to obtain the modified image shape Bx.

For example, the modification consisting in shifting an image shape is indicated with the character "a". Therefore, the modified image shape B obtained by shifting the image shape A, or by shifting the optionally defined second image shape C, is indicated as "Ba".

In one embodiment, the modification step 300 comprises shifting the imaging shape A.

In one embodiment, by shifting the imaging shape A it is meant to drag the image shape A in any direction in the image I so as to obtain a modified image shape Ba having the same proportions of the image shape A and having a different position compared to the position of the image shape A. Therefore, the image shape A and the modified image shape Ba are congruent.

By congruent image shapes, it is meant that the image shapes have the same shape and size. Two or more image shapes are congruent if they coincide precisely one with the other after one of the following modifications: flipping, shifting, rotating or a combination thereof.

One non-limiting example of this embodiment is illustrated in **figure 2****.** For intelligibility purpose, the images shapes A and Ba are represented within the image I as well as isolated from the parent image I.

In one embodiment, shifting the image shape A comprises: identifying the (x, y) coordinates of all the pixels of the image shape A; add a predetermined quantity x' to all the x coordinates and/or a predetermined quantity y' to all the y coordinates, so as to obtained a modified image shape Ba having the same shape as the image shape A and having a different position.

In one embodiment, said predetermined quantities x' and y' are comprised between 0 and x max, and between 0 and y max, respectively. X max and y max are selected so that, for each pixel of the image shape A having (x, y) coordinates, (x+x max, y+y max) do not exceed the dimensions of the image I.

In one embodiment, the modification step 300 comprises shifting the first image shape A by a shifting distance *a* in any direction, whilst preserving the size and the shape of the imaging shape A. The modified image shape Ba obtained in this embodiment has the same shape and a different position, when compared to the image shape A.

In one embodiment, by image shape position, it is meant the position of the center of the image shape in the image I.

In one embodiment, the method according to the present invention comprises a step of identifying the center of the image shape A.

In one embodiment, the center of the image shape A may be the symmetry center, the center of mass, the weighted center of mass, or the center of bounding box/ellipsoid.

In one embodiment, the magnitude of the shifting distance *a* is comprised between 0 and *a* max, wherein *a* max is the largest distance by which the image shape A can be shifted in a given direction while being entirely comprised within the boundaries of the image I.

In one embodiment, the shifting distance *a* is comprised between 0,0001% of *d* min and 100% of *d* min, wherein *d* min is the smallest diameter of the image shape A.

In one embodiment, a second image shape C is defined in the defining step 201, and the modification step 300 further comprises a modification step 301 of shifting the second image shape C.

In one embodiment, the terms dragged, shifted and displaced, referring to the image shape A or C, have the same meaning.

In one embodiment, the modification step 300 comprises resizing the image shape A.

In one embodiment, by resizing it is meant to apply a size multiplier b to the image shape A whilst preserving its position so as to obtain a modified image shape Bb that is similar to A.

By similar image shapes, it is meant that the image shapes have the same shape and different or equal size. Two or more image shapes are similar if they coincide precisely one with the other after one of the following modifications: resizing, flipping, shifting, rotating or a combination thereof.

In one embodiment, the multiplier b is inferior to 1. In this embodiment, the modified image shape Bb is a shrunk version of the image shape A.

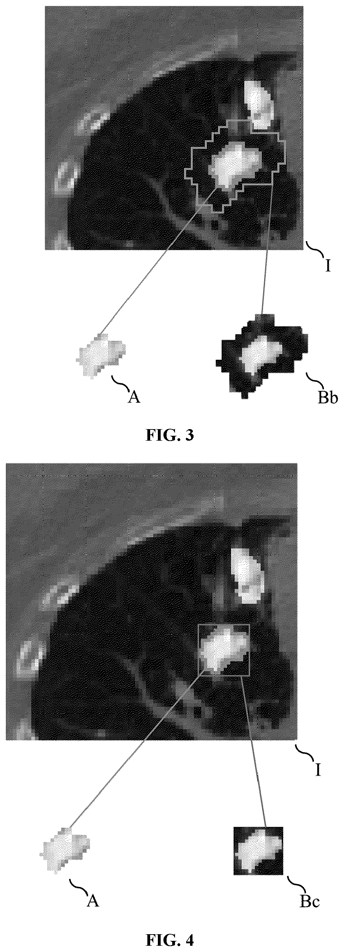

In one embodiment, the multiplier b is superior to 1. In this embodiment, the modified image shape Bb is a magnified version of the image shape A. One non-limiting example of this embodiment is illustrated in **figure 3****.** For intelligibility purpose, the images shapes A and Bb are represented within the image I as well as isolated from the image I.

In one embodiment, the multiplier b is equal to 1. In this embodiment, the modified image shape Bb and the image shape A are congruent.

In one embodiment, the multiplier *b* is comprised between 1 and *b* max, wherein *b* max is the largest multiplier for which none of the pixels of the modified image shape Bb extends outside the borders of a predefined area within the image I. In one embodiment, said predefined area within the image I is the whole image I, an area comprising the patient, or an area comprising an organ. In one embodiment, the predefined area is obtained via a region growing algorithm. In another embodiment, the predefined area is hand-drawn.

In one embodiment, the multiplier *b* is comprised between 0,25 and 1,75. Preferably, the multiplier *b* is comprised between 0,75 and 1,25.

In one embodiment, a second image shape C is defined in the defining step 201, and the modification step 300 further comprises a modification step 301 of resizing the second image shape C.

In one embodiment of the present invention, the modification step 300 comprises deforming the first imaging shape A.

In one embodiment, by deforming it is meant applying a deformation multiplier *c* so as to obtain a modified image shape Bc.

In one embodiment, by deforming it is meant displacing the vertexes of the image shape A to a target position.

In one embodiment, the deformation multiplier *c* is a geometrical deformation multiplier, *i.e.* a deformation multiplier that allows to obtain a modified image shape Bc that is a two-dimensional or three-dimensional geometrical shape. One non-limiting example of this embodiment, in which the modified image shape Bc is a square, is illustrated in **figure 4****.** For intelligibility purpose, the images shapes A and Bc are represented within the image I as well as isolated from the parent image I.

In this embodiment, the modification step 300 comprises:
- detecting in the image I a geometrical shape which closely fit the image shape A;
- for each pixel of the image shape A, calculating a geometrical multiplier *c* based on the detected geometrical shape;
- multiplying the position of each pixel of the image shape A for the geometrical multiplier *c,* so as to obtaining a modified image shape Bc.

In one embodiment, the geometrical shape is the obtained by means of a fitting criterion, *i.e.,* it is the geometrical shape that most closely fit the image shape A.

In one embodiment, the geometrical shape which closely fits the image shape A is a geometrical shape entirely comprised in the image shape A, such as for instance a polygon inscribed in the image shape A.

In one embodiment, the deformation multiplier c is a geometrical deformation multiplier that allows to obtain a modified image shape Bc that is entirely comprised within the image shape A, such as for instance a polygon inscribed in the image shape A.

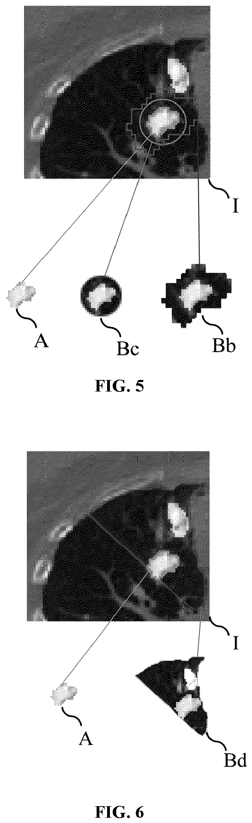

In one embodiment, the deformation multiplier *c* is a geometrical deformation multiplier that allows to obtain a modified image shape Bc in which the image shape A is entirely comprised. Non-limiting examples of this embodiment are illustrated in **figure 4** and **figure 5****.**

In one particular embodiment, the modified image shape Bc is a polygon circumscribed about the image shape A.

In one embodiment, the geometrical shape is a bidimensional shape, preferably a polygonal shape.

In one embodiment, the geometrical shape is a three-dimensional shape.

In one embodiment, the deformation multiplier is a physiological deformation multiplier *d.* By physiological deformation multiplier it is meant a multiplier that allows to obtain a modified image shape Bd that is similar to an organ, part of an organ, or an implanted structure within a human or animal body, or a cyborg.

In this embodiment, the modification step 300 comprises:
- detecting in the image I a physiological object;
- determining the shape of said physiological object;
- for each pixel of the image shape A, calculating a physiological deformation multiplier *d* based on the shape of the physiological object;
- multiplying the position of each pixel of the image shape A for the physiological deformation multiplier *d,* so as to obtaining a modified image shape Bd.

This embodiment ensures that the modified image shape Bd comprises relevant physiological areas so that image features are extracted not only from a lesion, but also from the surrounding tissues. This is all the more important in oncology, since tumor-surrounding tissues provide important information related to tumor classification, response to treatment, tumor progression or prognosis. Tumor-surrounding tissues may include edema, enhancing tumor, non-enhancing tumor, necrotic tissues, healthy tissues.

In one embodiment, the deformation multiplier *d* is a physiological deformation multiplier that allows to obtain a modified image shape Bd in which the image shape A is entirely comprised. One non-limiting example of this embodiment is illustrated in **figure 6****.**

In one embodiment, the deformation multiplier *d* is a physiological deformation multiplier that allows to obtain a modified image shape Bd that is entirely comprised within the image shape A.

For instance, the image shape A may be a region of interest corresponding to a suspected lung cancer. In this example, the modified image shapes Bd that are obtained with a physiological deformation multiplier *d* may include, but are not limited to: the lung comprising the suspected lung cancer, a necrotic area comprised within the suspected lung cancer.

In one embodiment, the physiological object in which the image shape A is entirely comprised is detected by means of one of the following: edge detection algorithm, region growing algorithm, image thresholding.

In one embodiment, the physiological object in which the image shape A is entirely comprised is a tissue, an organ, a portion of an organ, a patient.

In one embodiment, a second image shape C is defined in the defining step 201, and the modification step 300 further comprises a modification step 301 of deforming said second image shape C.

In one embodiment of the present invention, the modification step 300 comprises applying a morphological operation, such as erosion, dilatation, opening, closing or a combination thereof, to the image shape A.

In one embodiment, a second image shape C is defined in the defining step 201, and the modification step 300 further comprises a modification step 301 of applying a morphological operation to the second image shape C.

In one embodiment of the present invention, the modification step 300 comprises a modification such as rotation, reflection, affine transformation, polynomial transformation, or piecewise linear transformation.

In one embodiment, a second image shape C is defined in the defining step 201, and the modification step 300 comprises union, intersection, difference of the image shape A and the second image shape C, or a combination thereof.

In one embodiment, the modification step 300 comprises: union, intersection, difference of two or more modified image shapes B, or a combination thereof, so as to obtain additional modified image shapes B.

In the present method, the modification step 300 may comprise a combination of two or more modifications as described here above applied on the image shape A and/or the second image shape C and/or one or more modified image shapes B. The combined modifications may be applied in a succession.

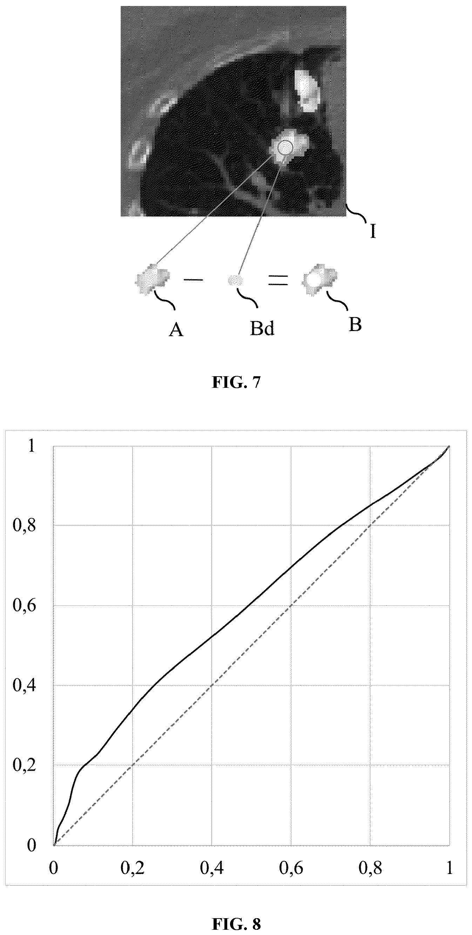

One non-limiting example of this embodiment is illustrated in **figure 7****.** In this particular example, the modified image shape B obtained via the modification step 300 is obtained via a combination of two modifications: a deformation applied on the image shape A, so as to obtain a modified image shape Bd; and a difference between said modified image shape Bd and the image shape A.

For intelligibility purpose, the images shapes A, Bd and B are represented within the image I as well as isolated from the parent image I.

The method according to the present invention comprises a step 400 for obtaining image feature parameters and image feature parameter values, which comprises:
- deriving a plurality of image feature parameters from each image shape defined (as obtained at step 200) and/or modified (as obtained at step 300); and
- calculating at least one image feature parameter value from each of the pluralities of image feature parameters.

The derivation step 400 allows to associate a plurality of image feature parameters to each region of interest previously defined and/or modified, hence to perform feature extraction.

In one embodiment, the derived image feature parameters comprise at least one shape feature, such as for instance: compactness, mean diameter, maximum diameter, minimum diameter, sphericity, surface, volume.

In one embodiment, the derived image feature parameters comprise at least one histogram statistics, such as for instance: 10^{th} percentile, 25^{th} percentile, 75^{th} percentile, 90^{th} percentile, entropy, intensity range, intensity variance, intensity standard deviation, kurtosis, mean absolute deviation, minimum intensity, maximum intensity, mean intensity, median intensity, root mean square, skewness, standard deviation, uniformity, variance.

In one embodiment, the image feature parameters comprise at least one texture features such as coarseness, contrast, correlation, energy, homogeneity, inverse difference moment, sum average, sum variance, sum entropy.

More precisely, a plurality of image feature parameters is derived from the image shape A and from each of the modified image shape B obtained in the modification step 300.

In one embodiment, a second region of interest is defined in the image I, so as to obtain a second image shape C. In this embodiment, the derivation step 400 comprises deriving a plurality of image feature parameters from the second image shape C.

In one particular embodiment, an image shape A and a second image shape C may be defined and modified so as to obtained two modified image shapes B, which are modified versions of A and C, such as for instance a shifted version of A and a resized version of C. In this particular embodiment, in the derivation step 400 a first plurality of image feature parameters is derived from the image shape A, a second plurality of image feature parameters is derived from the second image shape C, and a third and a fourth plurality of image feature parameters are derived from the two modified image shapes B.

From each of the pluralities of image feature parameters obtained in the derivation step 400, or selected in the selection step 500, at least one image feature parameter value is calculated. Since each plurality of image feature parameters is derived from a precise image shape A or B or C, the at least one image feature parameter value is a quantitative representation of said image shape.

The method according to the present invention comprises an optional step of selecting 500 a subset of image feature parameters from the pluralities of image feature parameters obtained in the derivation step 400, the subset of image feature parameters being selected based on the at least one image feature parameter value.

Some of the image feature parameters may be mutually correlated; moreover, the information related to different image feature parameters may not be equally relevant. The selection step 500 advantageously allows to reduce redundancy and to select the most relevant image feature parameters. As a result, in a predictive model built on said subset of image feature parameters the prediction error is minimized.

Said subset of image feature parameters comprises at least one image feature parameter derived from a modified image shape B.

In one embodiment, the subset of image feature parameters comprises all the image feature parameters derived from the plurality of modified image shapes B.

The feature selection step 500 allows to select the most relevant image feature parameters needed to build a prediction model. In one embodiment, the subset of image feature parameters is selected based on the predictive power of the model.

The subset of image feature parameters may be selected by means of an artificial intelligence feature selection technique.

Examples of artificial intelligence feature selection technique include, but are not limited to: a filter-based selection technique, a wrapper-based selection technique, an embedded method, or a combination thereof.

In one embodiment, the subset of image feature parameters comprises one image feature parameter derived from a modified image shape B.

In one embodiment, feature selection step 500 is optional. In this embodiment, all the image feature parameters obtained in the derivation step 400 are used to build a predictive model in the derivation step 600.

The method according to the present invention comprises a step of deriving 600 a predictive, diagnostic, therapeutic, prognostic or theragnostic value for supporting medical decision making, using reference data.

In the derivation step 600, a predictive value is derived by a processing unit based on the image feature parameters. In one embodiment, the value derived in derivation step 600 is a predictive value, a diagnostic value, a therapeutic value, a prognostic value or a theragnostic value.

In a first embodiment, a selection step 500 is performed to select a subset of image feature parameters, and the predictive value is derived based on said subset of image feature parameters. Selecting a subset of image feature parameters allows to minimize the error classification of the predictive model.

In a second embodiment, a selection step 500 is not performed, hence the predictive value is derived based on all the image feature parameters obtained in the derivation step 400.

The predictive value is derived based on at least one image feature parameter that is that is not exclusively obtained from the first image shape A, *i.e.,* it is derived from at least one image feature parameters obtained from the modified image shape B.

The modified image shapes B can capture information that is relevant for predictive and/or learning purposes; therefore, the performance of the predictive model is improved when the predictive value is derived based on at least one image feature parameter from modified image shape B.

In one embodiment, the predictive value is derived in the derivation step 600 by combining a plurality of image feature parameter values derived from the image A and from the modified image B.

In one embodiment, the predictive value is a linear combination of the image feature parameter values derived from the image A and from the modified image B wherein each image feature parameter value is multiplied by a weighting factor.

In another embodiment, the predictive value is a polynomial combination of the image feature parameter values derived from the image A and from the modified image B.

In one embodiment, the processing unit derives the predictive value using reference data. Reference data may comprise clinical outcomes, molecular markers, genetic markers.

In one embodiment, the derivation step 600 is performed by a machine learning algorithm running on a processing unit.

The present invention also relates to a system for performing image-based analysis tasks for providing information for supporting diagnostic, therapeutic, prognostic or theragnostic purposes of human and animal bodies.

In one embodiment, the system comprises a visualization module for displaying the image I, the image shape A, the plurality of modified image shapes B and, optionally, the second image shape C.

In one embodiment, the system comprises a processing unit the processing unit configured to run one or more rule-based algorithms or machine learning-based algorithms.

In one embodiment, the system according to the present invention comprises a processing unit capable of providing an image I.

In one embodiment, the processing unit is further configured to define regions of interest within the image I, so as to define an image shape A and, optionally, to define a second image shape C.

In one embodiment of the present system, the processing unit is configured to modify the first image shape A and, optionally, modify the second image shape C so as to obtain a plurality of modified image shapes B.

In one embodiment, the processing unit comprises a computation module configured to derive from each of the image shape A and the plurality of modified image shape B, a plurality of image feature parameters; and calculate at least one image feature parameter value from each plurality of image feature parameters.

In one embodiment, a second region of interest is defined in the image I, so as to obtain a second image shape C. In this embodiment, the computation module is further capable of:
- deriving a plurality of image features parameters associated with said second image shape C;
- calculating at least one image feature parameter value from the plurality of image features parameters associated with the second image shape C.

The processing unit may further be configured to run a selection algorithm configured to select a subset of image feature parameters from the pluralities of image feature parameters. In one embodiment, the selection algorithm is configured to select the subset of image feature parameters having a mutual correlation inferior to a predefined threshold.

In one embodiment, the processing unit is configured to run a machine learning algorithm to build a predictive model of a patient. In one embodiment, the output of the predictive model is a predictive, diagnostic, therapeutic, prognostic or theragnostic value for supporting medical decision making.

In one embodiment, the input of the predictive model comprises the pluralities of image feature parameters derived from each of the image shape A, the modified image shape B and optionally second image shape C.

In one embodiment, the input of the predictive model comprises a subset of image feature parameters selected from the pluralities of image feature parameters. Selecting a subset of image feature parameters allows to minimize the errors of the predictive model that are due to the presence of mutually correlated image feature parameters.

While various embodiments have been described and illustrated, the detailed description is not to be construed as being limited hereto. Various modifications can be made to the embodiments by those skilled in the art without departing from the true spirit and scope of the disclosure as defined by the claims.

The present invention also relates to a computer program product for image analysis, preferably for providing information for supporting diagnostic, therapeutic, prognostic or theragnostic purposes of human and animal bodies. The computer program product comprises instructions which, when the program is executed by a computer, cause the computer to carry out the steps of the method described hereabove.

The computer program according to the present invention allows to provide an automated image analysis method for providing clinically relevant information. This reduces user-dependent variations, thus providing a rapid and reliable support to the clinical decision-making process.

The computer program product to perform the method as described above may be written as computer programs, code segments, instructions or any combination thereof, for individually or collectively instructing or configuring the processor or computer to operate as a machine or special-purpose computer to perform the operations performed by hardware components. In one example, the computer program product includes machine code that is directly executed by a processor or a computer, such as machine code produced by a compiler. In another example, the computer program product includes higher-level code that is executed by a processor or a computer using an interpreter. Programmers of ordinary skill in the art can readily write the instructions or software based on the block diagrams and the flow charts illustrated in the drawings and the corresponding descriptions in the specification, which disclose algorithms for performing the operations of the method as described above.

The present invention also relates to a computer-readable storage medium comprising instructions which, when the program is executed by a computer, cause the computer to carry out the steps of the method according to any one of the embodiments described hereabove.

According to one embodiment, the computer-readable storage medium is a non-transitory computer-readable storage medium.

Computer programs implementing the method of the present embodiments can commonly be distributed to users on a distribution computer-readable storage medium such as, but not limited to, an SD card, an external storage device, a microchip, a flash memory device, a portable hard drive and software websites. From the distribution medium, the computer programs can be copied to a hard disk or a similar intermediate storage medium. The computer programs can be run by loading the computer instructions either from their distribution medium or their intermediate storage medium into the execution memory of the computer, configuring the computer to act in accordance with the method of this invention. All these operations are well- known to those skilled in the art of computer systems.

The instructions or software to control a processor or computer to implement the hardware components and perform the methods as described above, and any associated data, data files, and data structures, are recorded, stored, or fixed in or on one or more non-transitory computer-readable storage media. Examples of a non-transitory computer-readable storage medium include read-only memory (ROM), random-access memory (RAM), flash memory, CD- ROMs, CD- Rs, CD+ Rs, CD- RWs, CD+ RWs, DVD-ROMs, DVD- Rs, DVD+ Rs, DVD- RWs, DVD+ RWs, DVD- RAMs, BD- ROMs, BD- Rs, BD- R LTHs, BD- REs, magnetic tapes, floppy disks, magneto-optical data storage devices, optical data storage devices, hard disks, solid-state disks, and any device known to one of ordinary skill in the art that is capable of storing the instructions or software and any associated data, data files, and data structures in a non-transitory manner and providing the instructions or software and any associated data, data files, and data structures to a processor or computer so that the processor or computer can execute the instructions. In one example, the instructions or software and any associated data, data files, and data structures are distributed over network-coupled computer systems so that the instructions and software and any associated data, data files, and data structures are stored, accessed, and executed in a distributed fashion by the processor or computer.

While various embodiments have been described and illustrated, the detailed description is not to be construed as being limited hereto. Various modifications can be made to the embodiments by those skilled in the art without departing from the true spirit and scope of the disclosure as defined by the claims.

### DESCRIPTION OF THE DRAWINGS

Features and advantages of the invention will become apparent from the following description of embodiments of a system, this description being given merely by way of example and with reference to the appended drawings.
**Figure 1** represents an image I in which an ROI is defined so as to obtain an image shape A and wherein the image shape A is shifted so as to obtained a modified image shape Ba having the same shape and size as the image shape A and having a different position than the image shape A within the image I.
**Figure 2** represents an image I in which two ROIs are defined so as to obtain an image shape A and a second image shape C.
**Figure 3** represents an image I in which an ROI is defined so as to obtain an image shape A and wherein the image shape A is resized so as to obtained a modified image shape Bb being larger than the image shape A and having the same proportions as the image shape A.
**Figure 4** represents an image I in which an ROI is defined so as to obtain an image shape A and wherein the image shape A is deformed with a geometrical multiplier c so as to obtain a modified image shape Bc that is the closest square fitting to the image shape A.
**Figure 5** represents an image I in which an ROI is defined so as to obtain an image shape A and wherein a first and a second modifications are applied to said image shape A, so as to obtain two modified image shapes B; said first and second modifications comprise, respectively: deforming the image shape A with a geometrical multiplier c so as to obtain a first modified image shape Bc that is a circle comprising the image shape A, resizing the image shape A with size multiplier b so as to obtain a second modified image shape Bb.
**Figure 6** represents an image I in which an ROI is defined so as to obtain an image shape A and wherein the image shape A is deformed with a physiological multiplier d so as to obtain a modified image shape Bd that is a portion of the lung.
**Figure 7** represents an image I in which an ROI is defined so as to obtain an image shape A and wherein a combination of modifications is applied to the image shape A so as to obtain a modified image B, the combination of modification comprising: deforming the image shape A with a physiological multiplier *d* so as to obtain a modified image shape Bd that is the core of the tissue comprised in the image shape A; subtracting the image shape Bd thus obtained from the image shape A so as to obtained a modified image shape B.
**Figure 8** is a graph representing the receiver operator curve (black continuous line) of an exemplary predictive model obtained with the method of the present invention, in which the predictive value is obtained on the basis of at least one image feature parameter derived from a modified image shape B.
**Figure 9** is a graph representing the receiver operator curve (black continuous line) of a predictive model obtained with a method of the prior art, in which the predictive value is obtained on the basis of image feature parameters derived only from the image shapes A.

### EXAMPLE

Tumor histological subtype is one of the main clinical aspects that may influence treatment decision making for non-small cell lung cancer (NSCLC) patients. In the following example, the present invention is applied to evaluate the performance of a machine learning model to classify the squamous cell carcinoma (SCC) histological subtype of NSCLC. In this example, the predictive value is derived by means of a machine learning algorithm.

### Materials and Methods

CT-images from 266 patients harboring a pathologically-confirmed NSCLC, and with confirmed histological subtype, were obtained from the open-source dataset LUNG1, which comprises images from 422 NSCLC patients. The corresponding histological subtypes are provided in Table 1.

**Table 1**

| Histological subtype | Number of patients |
|---|---|
| Adenocarcinoma | 51 |
| Large cell carcinoma | 114 |
| Squamous cell carcinoma | 152 |
| Not otherwise specified | 63 |
| Not available | 42 |

CT-images from all of the 266 cases with a clearly defined histological subtype were included in the present study.

On each CT-image, a region of interest (ROI) around the gross tumor volume was manually delineated by an expert to obtain an image shape A. Subsequently, the ROIs were modified, so as to obtain the following 6 modified image shapes B, from each image shape A. The modified image shapes thus obtained are described in detail hereunder.
- Image shape B1 is obtained by applying a geometrical deformation multiplier, so as to obtain the circumscribed sphere of the image shape A which most closely fits around the image shape A.
- Image shape B2 is obtained by applying a geometrical deformation multiplier, so as to obtain the inscribed sphere of the image shape A which most closely fits inside the image shape A.
- Image shape B3 is obtained via a combination of two modifications. First a resizing multiplier is applied to image shape A, so as to obtain a modified image shape B3a, which is a 10 mm shrinkage of the image shape A in all directions. The second modification is the difference between the image shape A and the image shape B3a; so as to obtain a modified image shape B3 which is an inner shell with a thickness of 10 mm.
- Image shape B4 is also obtained via a combination of two modifications. First a resizing multiplier is applied to image shape A, so as to obtain a modified image shape B4a, which is a 10 mm enlargement of the image shape A in all directions. The second modification is the difference between the image shape B4a and the image shape A; so as to obtain a modified image shape B4 which is an outer shell with a thickness of 10 mm.
- Image shape B5 is obtained by applying a resizing multiplier, so as to obtain a modified image shape B5, which is a 10 mm enlargement of the image shape A in all directions.
- Image shape B6 is obtained by applying a resizing multiplier, so as to obtain a modified image shape B5 which is a 3 mm shrinkage in all directions.

For each of the image shapes A, B1, B2, B3, B4, B5, and B6, 172 image feature parameters were derived. The extracted features comprised first order statistics, texture, and shape.

Multivariable logistic regression with Elastic Net regularization was performed according to the following methodology. Highly correlated features, features with near zero variance and linear combinations between features were first eliminated from further analysis. For each highly correlated feature pair (Pearson correlation coefficient p > 0.8), the variable with the largest mean absolute correlation with all remaining features was removed. Model training was performed using 10 times repeated 10-fold cross-validation to select the optimal model hyperparameters, optimizing for the area under the receiver operator curve (AUC). Overall out of sample model performance was then evaluated as the mean repeated cross validation AUC for the model with optimal hyperparameters.

In the derivation step, a first predictive model was built to provide a first predictive value for classification of SCC on the basis of a first subset of image feature parameters comprising image feature parameters derived from the image shapes A and the modified image shapes B1 to B6.

A second predictive model was further built to provide a second predictive value for classification of SCC, said second predictive value being derived on the basis of a second subset of image feature parameters including image feature parameters exclusively derived from the image shapes A.

### Results

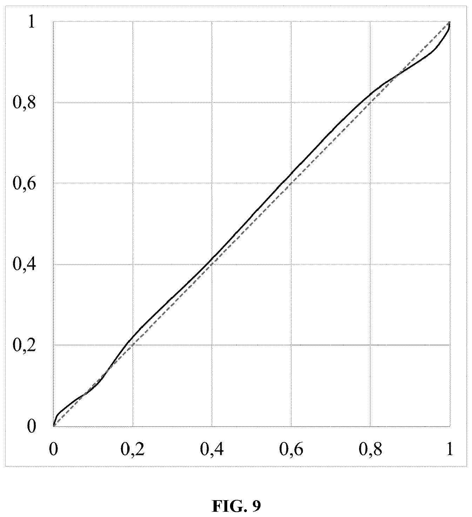

The receiver operator curves of the first and second models are shown in figures 8 and 9, respectively (horizontal axis: false positive rate, vertical axis: true positive rate, (gray dotted line: chance line). The area under the curve (AUC) for the first model, which comprises additional information contained in the modified image ROI shapes B1-B6, was 0.61. The AUC for the second model, which includes only feature parameters derived from image shape A, was 0.52. The AUC of the first model was statistically significantly higher than the AUC of the first model (H0: equal AUC; p < 0.05).

### Conclusion

These results show that the performance of the model is significantly improved when the predictive value is derived based on at least one image feature parameter from the modified image shapes B.

## Claims

1. Image analysis method for providing information for supporting diagnostic, therapeutic, prognostic or theragnostic purposes of human and animal bodies, comprising the steps of:
- providing (100), by a processing unit, an image I;
- defining (200), by said processing unit, a region of interest to obtain a first image shape A and optionally defining (201), by said processing unit, a second region of interest to obtain a second image shape C;
- modifying (300), by said processing unit, the first image shape A and optionally modifying (301) the second image shape C so as to obtain a plurality of modified image shapes B;
- deriving (400), by said processing unit, for each of the image shapes defined in the defining step (200, 201) and obtained in the modifying step (300, 301), a plurality of image feature parameters; and calculating at least one image feature parameter value from each plurality of image feature parameters;
- optionally selecting (500), by said processing unit, a subset of image feature parameters from the pluralities of image feature parameters obtained in the derivation step, the subset of image feature parameters being selected based on the at least one image feature parameter value;
- deriving (600), by said processing unit, using reference data a predictive, diagnostic, therapeutic, prognostic or theragnostic value for supporting medical decision making,
wherein the predictive value is derived based on at least one image feature parameter that is derived from the modified image shape B.

2. The image analysis method according to claim **1,** whereas the modification step (300) comprises shifting of the center of the first imaging shape A and optionally shifting the center of the second image shape C.

3. The image analysis method according to claim **2,** wherein the modified image shape B is obtained by shifting the center of the first image shape A by a shifting distance *a* in any direction a whilst preserving the size and the shape of the image shape A, so as to obtain a modified image shape Ba.

4. The image analysis method according to claim **3,** wherein the shifting distance *a* is comprised between 0 and *a* max, wherein *a* max is the largest shift in a specified direction without any part of the modified image shape Ba extending outside the image I region.

5. The image analysis method according to any of claim **1** to **4,** wherein the modification step (300) comprises resizing the first image shape A and optionally resizing the second image shape C.

6. The image analysis method according to claim **5,** wherein the resizing is performed by applying a size multiplier *b* whilst preserving the center position, so as to obtain a modified image shape Bb.

7. The image analysis method according to claim **6,** wherein the resizing multiplier b is > 0 and less than *b* max, where *b* max is the largest multiplier for which none of the modified image shape Bb extends outside the image region.

8. The image analysis method according to any of the claim **1** to 7, wherein the modification step (300) comprises deforming the first image shape A and optionally deforming the second image shape C.

9. The image analysis method according to claim **8,** wherein the deforming is performed by applying a geometrical deformation multiplier *c* to obtain a modified image shape Bc.

10. The image analysis method according to claim **9** wherein the geometrical deformation multiplier *c* is selected based on the two-dimensional or three-dimensional geometrical shape most closely fitting to the image shape A.

11. The image analysis method according to claim **8,** wherein the modification step (300) comprises deforming the first imaging shape A and optionally deforming the second image shape C by a physiological deformation multiplier *d* to obtain a modified image shape Bd.

12. The image analysis method according to claim **11,** wherein the physiological deformation multiplier *d* is selected based on the shape and size of an organ or parts thereof.

13. The image analysis method according to any of claims **1** to **12,** wherein the modification step (300) comprises two or more of the shifting, resizing or deformation steps according to any one of claims **2** to **12.**

14. The image analysis method according to any of claims **1** to **13,** wherein the modification step (300) comprises at least one morphological operation between two or more of any of the available shape A, shape C and shapes B.

15. The image analysis method according to any of claims **1** to **14,** wherein the derivation step (600) comprises: combining a plurality of image feature parameter values derived from the first image A and from the modified image B.

16. System for performing image-based analysis tasks for providing information for supporting diagnostic, therapeutic, prognostic or theragnostic purposes of human and animal bodies, the system comprising a processing unit configured to:
- provide an image I;
- define a region of interest in the image I so as to obtain a first image shape A and optionally define a second region of interest so to obtain a second image shape C;
- modify the first image shape A and optionally modify the second image shape C to obtain a plurality of modified image shapes B;
- derive from each of the image shape A, the modified image shape B and optionally second image shape C, a plurality of image feature parameters; and calculate at least one image feature parameter value from each plurality of image feature parameters;
- based on the at least one image feature parameter value, select a subset of image feature parameters from the pluralities of image feature parameters, the subset of image feature parameters comprising at least one image features parameter derived from the modified image shape B;
- using reference data, derive a predictive, diagnostic, therapeutic, prognostic or theragnostic value for supporting medical decision making.

17. A computer program product for providing information supporting diagnostic, therapeutic, prognostic or theragnostic purposes of human and/or animal bodies, the computer program product comprising instructions which, when the program is executed by a computer, cause the computer to carry out the steps of the method according to any of claims **1** to **15.**

18. A computer-readable storage medium comprising instructions which, when the program is executed by a computer, cause the computer to carry out the steps of the method according to any of claims **1** to **15.**
